# EUROPEAN PATENT APPLICATION

(11) **EP 4 595 888 A1**
(43) Date of publication of application: **06.08.2025**
(21) Application number: 23871338.2
(22) Date of filing: 26.06.2023
(51) Int. Cl.: A61B 6/03, A61N 5/10

(54) **IRRADIATION POSITION VERIFICATION ASSISTING DEVICE, IRRADIATION POSITION VERIFICATION ASSISTING METHOD, AND IRRADIATION POSITION VERIFICATION ASSISTING PROGRAM**

(30) Priority: 27.09.2022 JP 2022154021
(71) Applicant: HITACHI HIGH-TECH CORPORATION, Tokyo 105-6409 (JP)
(72) Inventor: YOSHIDA Hanae, Tokyo 100-8280 (JP); LI Yun, Tokyo 100-8280 (JP); UMEKAWA Toru, Tokyo 100-8280 (JP)
(74) Representative: MERH-IP Matias Erny Reichl Hoffmann Patentanwälte PartG mbB
(86) International application number: PCT/JP2023/023631
(87) International publication number: WO 2024/070093

(57) **Abstract**

This irradiation position verification assisting device includes an acquisition unit that acquires a first medical image of the inside of a subject's body, a second medical image of the inside of the subject's body captured after the first medical image has been captured, and a third medical image of the inside of the subject's body captured after the second medical image has been captured; a region analysis unit that analyzes a region contained in the first medical image; an association analysis unit that calculates first change information indicating a change related to the same tissue by aligning the first medical image with the second medical image, and transforms the region analysis result based on the first change information to output the transformed region analysis result; and a generation unit that superimposes the transformed region analysis result on the third medical image to generate a superimposed image.

## Description

### Cross-Reference to Related Application

The present application claims the priority of Japanese Patent Application No. 2022-154021, filed on September 27, 2022, the entire contents of which are incorporated herein by reference.

### Technical Field

The present invention relates to an irradiated position confirmation support device, an irradiated position confirmation support method, and an irradiated position confirmation support program for supporting confirmation of an irradiated position.

### Background Art

Stereotactic irradiation (SRT), which has recently become the mainstream in radiotherapy, is a technique of concentratedly irradiating a treatment target site with radiation rays from a plurality of angles. This reduces the impact on normal tissue and cells, enabling precise irradiation with radiation rays on the treatment target site.

In addition to this, intensity modulated radiation therapy (IMRT), which is capable of changing the intensity of radiation rays to be irradiated, has also been developed. This enables irradiation on a pinpoint with radiation rays even for a tumor in complicated contact with normal cells.

These functions have been installed as standard in recent radiotherapy devices, and development has also been advanced for image-guided radiotherapy (IGRT) as an essential technique for SRT and IMRT. IGRT is a technique for accurately performing treatment while correcting a position error during radiotherapy with reference to image information on the subject (X-ray image or the like) obtained immediately before and during irradiation.

SRT, IMRT, and IGRT can concentrated radiation rays on a tumor and reduce exposure to healthy cells, but still have particular challenges in application to trunk tumors such as lung cancer. The position of lung cancer also changes during irradiation of treatment rays due to breathing, and it is said that the movement distance at this time reaches up to several tens of millimeters even in about one second. To reliably administer the radiation rays to a treatment target site accompanied by such respiratory movement, the irradiation needs to be performed while assuming the movement of the tumor.

The related art has adopted a method for reliably irradiating an actual treatment target region by setting, as the irradiation region, a region acquired by adding an assumed movement range as a margin to the treatment target region. Unfortunately, this method increases the amount of exposure to normal tissue.

On the other hand, there is also a breath stop method in which the subject temporarily stops breathing, which imposes a heavy burden on the subject. As a method for simultaneously reducing the exposure to normal tissue and the burden on the subject as much as possible, there is moving body tracking irradiation. Various methods using moving body tracking irradiation have been developed. Examples thereof include: a method of measuring the movement of the body surface with an infrared ray or the like and estimating the tumor position based on the measurement; a method of measuring and tracking a metal marker pierced into the vicinity of the tumor by X-ray fluoroscopy; and further, a method of performing real-time tracking based on accurate measurement of tumor dynamics (position change, deformation, or the like) without a marker.

In any of the above methods using moving body tracking irradiation, when irradiating with treatment rays, irradiation is semi-automatically performed on the identified tumor position using the angle and the dose set in the treatment plan. Unfortunately, the identified treatment target range is not necessarily accurate. In order to avoid irradiation outside the treatment target range, it is necessary to confirm that the tumor can be correctly tracked by the X-ray fluoroscopic video at the time of irradiation in actual treatment.

The X-ray fluoroscopic image is characterized in that regions having a higher radiation absorption rate are delineated among objects on the same irradiation ray. Therefore, in such a confirmation screen, depending on the position and movement of the tumor, the tumor itself may be not hidden by another tissue without being rendered on the X-ray fluoroscopic image. This may lead to a variation in determination for each doctor, or may lead to hesitation in determination in treatment involving immediate determination. This point of view is considered as a very large problem in the moving body tracking function.

Existing techniques for solving this problem include the following PTL 1 and PTL 2. A radiotherapy system of PTL 1 includes a bed for placing a subject, a bed positioning device for positioning a bed in radiotherapy, and an X-ray imager including an X-ray generator for generating X-rays and an X-ray receiver for receiving X-rays from the X-ray generator. The bed positioning device generates bed positioning data based on first X-ray fluoroscopic image data captured by the X-ray imager and soft tissue projection image data generated from X-ray CT image data acquired at the time of treatment planning.

PTL 2 discloses a medical image diagnostic device that superimposes information obtained by analyzing medical image data generated by a modality different from the host device on a medical image generated by the host device. The medical image diagnostic device includes: an acquisition unit for acquiring at least one piece of analysis information obtained by analyzing medical image data on a subject generated by another medical image diagnostic device, which is a modality different from the host device; and a generation unit for generating, and displaying on a display, a superimposed image obtained by superimposing an image indicating the at least one piece of analysis information and a first medical image of the subject generated by the host device.

### Citation List

### Patent Literature

PTL 1: JP2011-072457A
PTL 2: JP2017-113312A

### Summary of Invention

### Technical Problem

In the existing techniques described above, it may be difficult to accurately present region information on a tumor or a tissue on a two-dimensional irradiated position confirmation screen in response to the change of the subject between different time points in units of several days, such as between the time of treatment planning and the time of treatment.

An object of the present invention is to improve the accuracy in confirming an irradiated position.

### Solution to Problem

An irradiated position confirmation support device as an aspect of the invention disclosed in the present application includes: an acquisition unit configured to acquire a first medical image acquired by imaging an inside of a body of a subject, a second medical image acquired by imaging the inside of the body of the subject after imaging the first medical image, and a third medical image acquired by imaging the inside of the body of the subject after imaging the second medical image; a region analysis unit configured to analyze a region included in the first medical image acquired by the acquisition unit; a relation analysis unit configured to calculate first change information indicating a change related to the same tissue in the first medical image and the second medical image by registering the first medical image with the second medical image acquired by the acquisition unit, and to output a deformed region analysis result by deforming a region analysis result acquired by the region analysis unit based on the first change information; and a generation unit configured to generate a superimposed image by superimposing the deformed region analysis result acquired by the relation analysis unit on the third medical image acquired by the acquisition unit.

### Advantageous Effects of Invention

According to the representative embodiment of the present invention, it is possible to improve the accuracy in confirming an irradiated position. Problems, configurations, and effects other than those described above will be clarified by descriptions of the following embodiments.

### Brief Description of Drawings

[FIG. 1] FIG. 1 is a block diagram illustrating a functional configuration example of an irradiated position confirmation support system.
[FIG. 2] FIG. 2 is a flowchart illustrating an example of an irradiated position confirmation support procedure by the irradiated position confirmation support device.
[FIG. 3] FIG. 3 is a diagram illustrating a display example of a superimposed image.
[FIG. 4] FIG. 4 is a diagram illustrating an example of a contour setting table.
[FIG. 5] FIG. 5 is a diagram illustrating the inside of a human body.
[FIG. 6] FIG. 6 is a diagram illustrating Registration Example 1..
[FIG. 7] FIG. 7 is a diagram illustrating Registration Example 2.
[FIG. 8] FIG. 8 is a diagram illustrating Registration Example 3.
[FIG. 9] FIG. 9 is a diagram illustrating Registration Example 4.
[FIG. 10] FIG. 10 is a diagram illustrating a display example of a monitor.
[FIG. 11] FIG. 11 is a block diagram illustrating a hardware structure example of an irradiated position confirmation support system.

### Description of Embodiments

The present embodiment will describe an example of an irradiated position confirmation support system capable of supporting the confirmation of the validity of estimation when presenting an estimated position of a lesion that is invisible or difficult to see due to the angle or the situation on an irradiated position confirmation screen at the time of moving body tracking irradiation.

### <Functional Configuration Example of Irradiated Position Confirmation Support System>

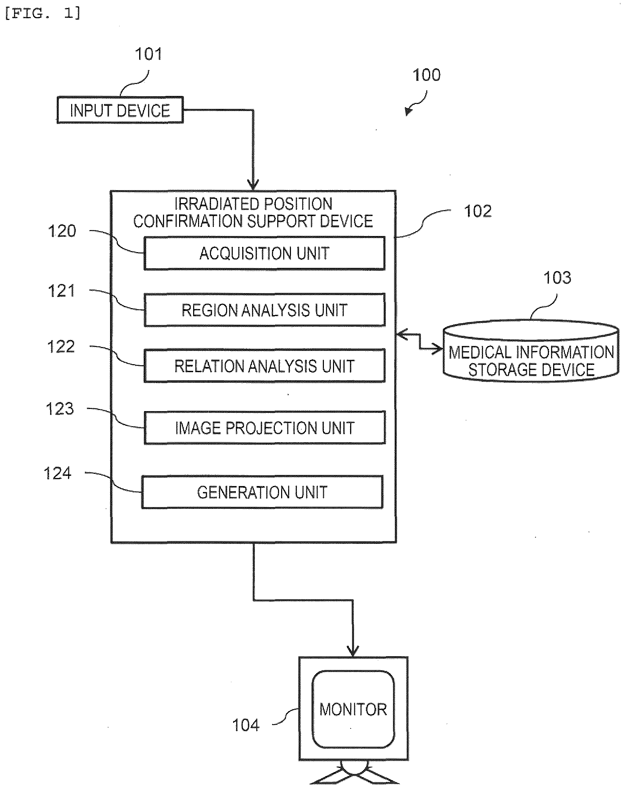

FIG. 1 is a block diagram illustrating a functional configuration example of an irradiated position confirmation support system. The irradiated position confirmation support system 100 includes an input device 101, an irradiated position confirmation support device 102, a medical information storage device 103, and a monitor 104.

The input device 101 receives data from the operator or the higher system and transmits the data to the irradiated position confirmation support device 102. The medical information storage device 103 stores various data such as medical images and other medical information. The monitor 104 displays the medical images and other medical information output from the irradiated position confirmation support device 102.

The irradiated position confirmation support device 102 is connected to the input device 101, the medical information storage device 103, and the monitor 104. The irradiated position confirmation support device 102 accesses the medical information storage device 103, reads data from the medical information storage device 103, and writes data to the medical information storage device 103.

The irradiated position confirmation support device 102 includes an acquisition unit 120, a region analysis unit 121, a relation analysis unit 122, an image projection unit 123, and a generation unit 124.

The acquisition unit 120 acquires data output from the input device 101 and stores the data in the medical information storage device 103. The data output from the input device 101 includes, for example, medical images such as a planning-time computed tomography (CT) image, a pre-treatment cone beam computed tomography (CBCT) image, and an in-treatment confirmation image.

The planning-time CT image is a CT image of the subject captured at a planning stage several days before treatment using a radiotherapy device. The pre-treatment CBCT image is, for example, a CBCT image captured for positioning the subject before starting the treatment. The in-treatment confirmation image is an image of the subject captured during the treatment, and may be a video or an X-ray fluoroscopic measurement video captured from two or more directions.

The region analysis unit 121 performs region analysis on the planning-time CT image. The region analysis is, for example, a process of specifying the regions of the tumor and a tissue such as the peripheral organs (lung, heart, spine, esophagus, or the like if the planning-time CT image is an image of the chest) based on three-dimensional CT volume data such as the planning-time CT image. The planning-time CT image after the region analysis by the region analysis unit 121 is referred to as a planning-time region analysis result. For example, the region analysis unit 121 assigns a character string indicating the name of the tissue to the region of the specified tissue (hereinafter referred to as a region name) in the planning-time region analysis result.

The relation analysis unit 122 registers the medical images. Specifically, for example, the relation analysis unit 122 compares the planning-time CT image with the pre-treatment CBCT image, and registers the positions of the objects included in the respective images. For example, the relation analysis unit 122 moves the planning-time CT image to match the pre-treatment CBCT image, and calculates first change information as the result of the registration.

The relation analysis unit 122 deforms the planning-time region analysis result using the first change information to obtain a pre-treatment CBCT image analysis result. The pre-treatment CBCT image analysis result is assumed to be equivalent to the region analysis result acquired by performing the region analysis on the pre-treatment CBCT image by the region analysis unit 121.

The relation analysis unit 122 registers the projected medical image and the in-treatment confirmation image, and calculates second change information for moving the projected medical image in accordance with the in-treatment confirmation image.

The relation analysis unit 122 deforms the projection analysis result generated by the image projection unit 123 using the second change information, and outputs a deformed projection analysis result.

The image projection unit 123 projects a three-dimensional image or three-dimensional region information onto a two-dimensional plane. Specifically, for example, the image projection unit 123 generates a two-dimensional projected medical image by projecting the pre-treatment CBCT image, which is a three-dimensional image, onto a two-dimensional plane using projection conditions set in advance.

The projection condition includes an X-ray irradiation angle (in-treatment confirmation angle) for creating an X-ray fluoroscopic measurement image displayed on the confirmation screen during treatment (in-treatment confirmation image). The projected medical image is a two-dimensional image simulating an X-ray fluoroscopic measurement image when X-rays are emitted from the same angle as that of the in-treatment confirmation image.

The image projection unit 123 generates a two-dimensional projection analysis result by projecting the pre-treatment CBCT image analysis result obtained from the relation analysis unit 122 on the two-dimensional plane based on the two-dimensional conversion information. The two-dimensional conversion information is the projection conditions, that is, information that includes the projection direction and is for converting the pre-treatment CBCT image analysis result, which is three-dimensional region information, into two pieces of two-dimensional region information (for example, volume rendering).

The generation unit 124 generates a superimposed image obtained by superimposing two medical images and outputs the superimposed image to the monitor 104. For example, the generation unit 124 generates a superimposed image of the deformed projection analysis result and the in-treatment confirmation image.

### <Irradiated Position Confirmation Support Procedure>

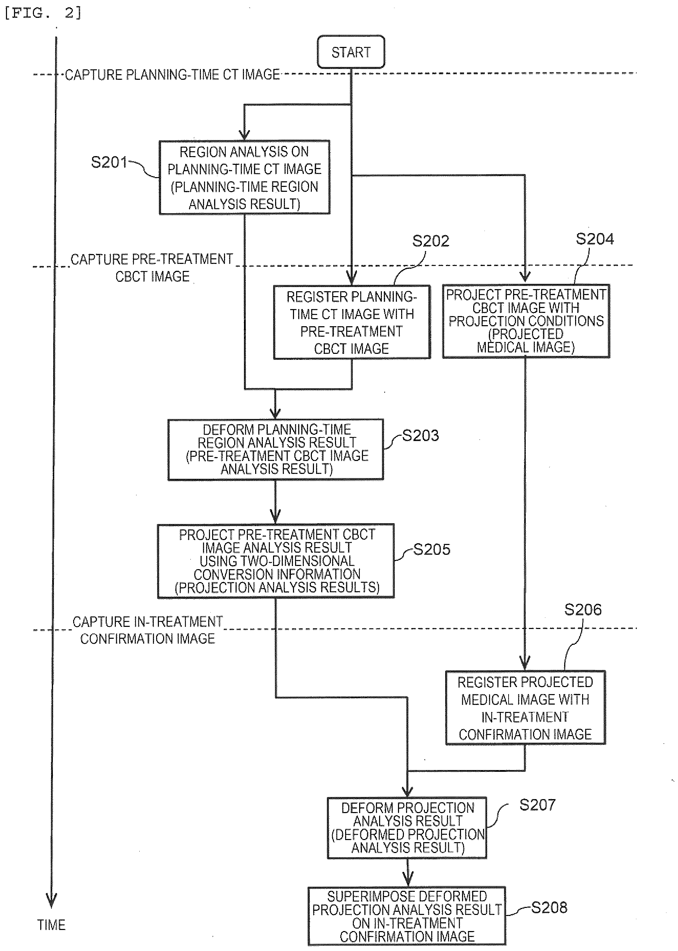

FIG. 2 is a flowchart illustrating an example of an irradiated position confirmation support procedure by the irradiated position confirmation support device 102. In FIG. 2, the timing for executing each step is clearly indicated by a timeline specifying various imaging timings.

After the planning-time CT image is captured and before the pre-treatment CBCT image is captured, the region analysis unit 121 generates the planning-time region analysis result by performing region analysis on the planning-time CT image according to an instruction from the higher system or the operator (step S201). The region analysis unit 121 stores the planning-time region analysis result in the medical information storage device 103.

After capturing the pre-treatment CBCT image and before capturing the in-treatment confirmation image, the relation analysis unit 122 registers the planning-time CT image with the pre-treatment CBCT image and calculates the first change information as a result of the registration (step S202). The relation analysis unit 122 stores the first change information in the medical information storage device 103.

After capturing the pre-treatment CBCT image and before capturing the in-treatment confirmation image, the relation analysis unit 122 deforms the planning-time region analysis result generated in step S201 using the first change information calculated in step S202 to generate the pre-treatment CBCT image analysis result (step S203). This pre-treatment CBCT image analysis result is assumed to be equivalent to the region analysis result acquired by performing the region analysis on the pre-treatment CBCT image by the region analysis unit 121. The relation analysis unit 122 stores the pre-treatment CBCT image analysis result in the medical information storage device 103.

After capturing the pre-treatment CBCT image and before capturing the in-treatment confirmation image, the image projection unit 123 generates a two-dimensional projected medical image by projecting the pre-treatment CBCT image onto a two-dimensional plane using the projection conditions set in advance (step S204). The image projection unit 123 stores the projected medical image in the medical information storage device 103.

After capturing the pre-treatment CBCT image and before capturing the in-treatment confirmation image, the image projection unit 123 generates a two-dimensional projected medical image as a projection analysis result by projecting the pre-treatment CBCT image analysis result onto a two-dimensional plane based on the two-dimensional conversion information (step S205). The image projection unit 123 stores the projected medical image in the medical information storage device 103.

After capturing the in-treatment confirmation image, the relation analysis unit 122 registers the projected medical image and the in-treatment confirmation image in the same manner as in step S202, and calculates second change information for moving the projected medical image in accordance with the in-treatment confirmation image (step S206). The second change information is also a vector for each coordinate value such as the first change information. The relation analysis unit 122 stores the second change information in the medical information storage device 103.

After capturing the in-treatment confirmation image, the relation analysis unit 122 deforms the projection analysis result using the second change information, and outputs the deformed projection analysis result (step S207). This deformed projection analysis result is assumed to be equivalent to the region analysis result acquired by performing the region analysis on the in-treatment confirmation image by the region analysis unit 121.

After capturing the in-treatment confirmation image, the generation unit 124 generates a superimposed image by superimposing the deformed projection analysis result on the in-treatment confirmation image, and outputs the superimposed image to the monitor 104 (step S208). Thus, the superimposed image is displayed on the monitor 104.

### <Display Example of Superimposed Image>

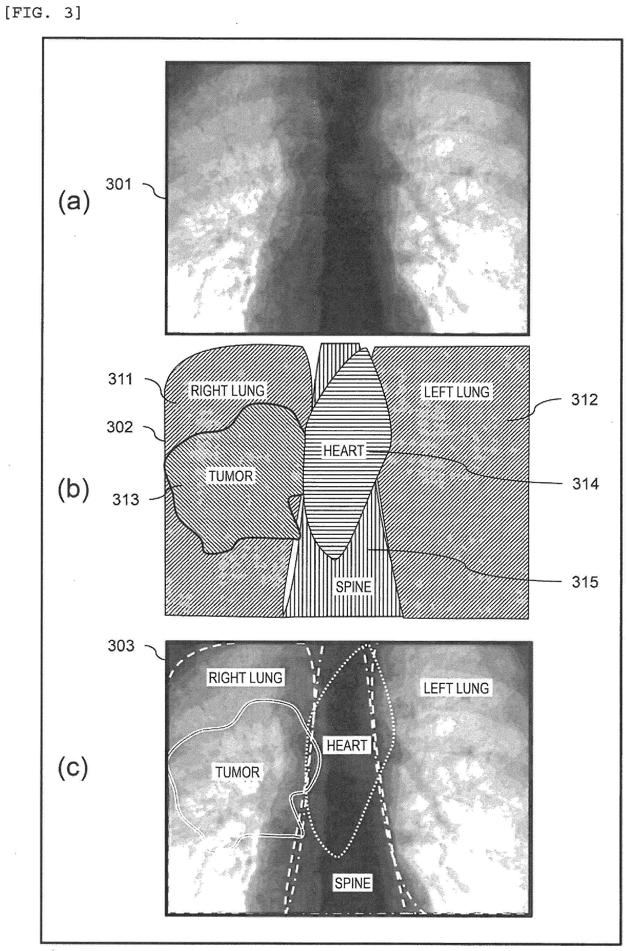

FIG. 3 is a diagram illustrating a display example of a superimposed image. (a) is an in-treatment confirmation image 301 used in step S206. (b) is a deformed projection analysis result 302 acquired by deforming the region to match the in-treatment confirmation image 301 by the relation analysis unit 122 in step S207. (c) illustrates the superimposed image 303 superimposed in step S208.

The in-treatment confirmation image 301 and the deformed projection analysis result 302 are images of the chest of the same subject. Since the region analysis is not executed on the in-treatment confirmation image 301 of (a) by the region analysis unit 121, the contour and the region name of the region are not specified.

On the other hand, in the deformed projection analysis result 302 in (b), region names (a right lung 311, a left lung 312, a tumor 313, a heart 314, and a spine 315) are already assigned to the regions together with the contours in the planning-time region analysis result acquired by the region analysis (step S201) in the region analysis unit 121.

The regions are not overlapped for the sake of convenience in (b), but may be actually overlapped. As illustrated in (c), the generation unit 124 superimposes the contours (indicated by white dotted lines, broken lines, chain lines, and double lines) and the region names of the regions (the right lung 311, the left lung 312, the tumor 313, the heart 314, and the spine 315) of the deformed projection analysis result 302 on the in-treatment confirmation image 301. The setting of the contours may be defined in advance according to the region.

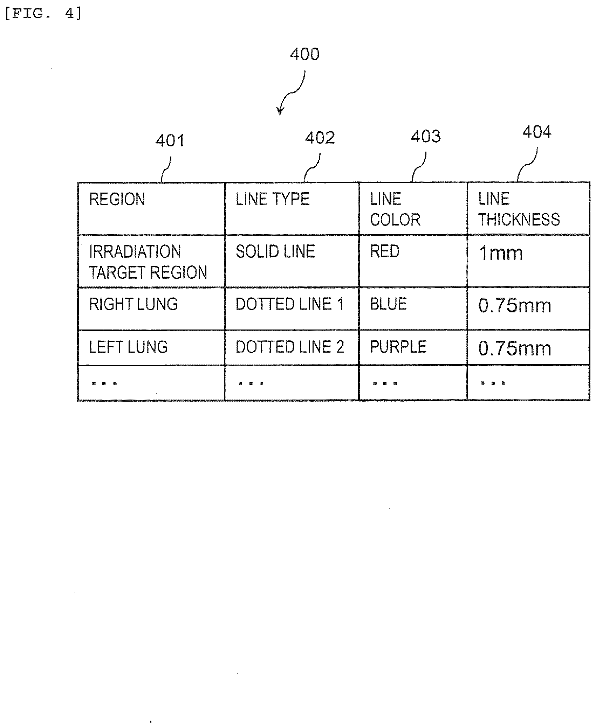

FIG. 4 is a diagram illustrating an example of the contour setting table. The contour setting table 400 is stored in the medical information storage device 103. The contour setting table 400 includes, as fields, a region 401, a line type 402, a line color 403, and a line thickness 404.

The regions 401 include the right lung 311, the left lung 312, the tumor 313, the heart 314, the spine 315, and the irradiation target region for the moving body tracking irradiation. The line type 402 distinguishes the drawing format of the contour for each region 401, such as a dotted line, a broken line, a chain line, or a double line. The line color 403 distinguishes the contour color for each region 401, such as red, blue, or purple. The line thickness 404 distinguishes the contour thickness for each region 401, such as 1 mm or 0.75 mm.

FIGS. 3 and 4 illustrate an example in which the contours of the regions 401 are superimposed, but the information to be superimposed here is not limited to the contours, and for example, a method of superimposing the regions 401 in a translucent fill representation may be adopted. Also in this case, the regions 401 can be displayed in a manner easy to understand by utilizing not only monochrome but also color representation. However, in the case of the fill representation, since the color information is also overwritten on the original in-treatment confirmation image 301, the representation of the regions 401 is designed according to the user's preference or designed such that the in-treatment confirmation image 301 itself is not difficult to view.

### <Projection Method>

The following will describe a method for the image projection unit 123 to project the three-dimensional pre-treatment CBCT image analysis result as a two-dimensional projection analysis result using the two-dimensional conversion information.

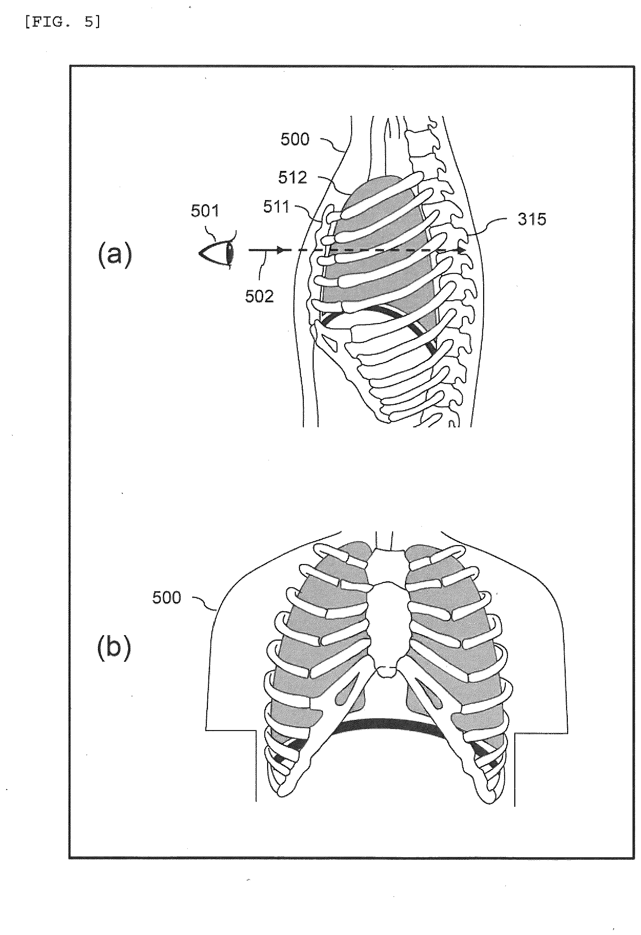

FIG. 5 is a diagram illustrating the inside of a human body. (a) is a partial cross-sectional side view of a human body 500 of the subject. In (a), assuming that an eye 501 of another person is in front of the human body 500 and the human body 500 is in the visual line direction of the eye 501, the position of the eye 501 is an X-ray irradiated position to be irradiated with X-rays. (b) is a partial front cross-sectional view of the human body 500 of the subject. (b) is a diagram in which the three-dimensional position correlation is correctly made two-dimensional (except for the skin).

That is, assuming that a visual line direction 502 from the position of the eye 501 in (a) is the projection direction, organic tissues are present inside the human body 500 in the order of ribs 511, lungs 512 (the right lung 311 and the left lung 312), and the spine 315. Therefore, a part of the lungs 512 and most of the spine 315 are in front of these (closer to the start point of the arrow indicating the visual line direction 502), and thus are not delineated.

Incidentally, in the case of the X-ray fluoroscopic measurement image, an object having a higher radiation absorption rate is preferentially delineated when viewed from the visual line direction 502 regardless of the three-dimensional position correlation. Therefore, an object having a lower radiation absorption rate on the same irradiation ray as the object having a higher radiation absorption rate is not delineated. Since bones such as the ribs 511 and the spine 315 are objects having a higher radiation absorption rate, according to the principle of the radiation absorption rate, if the tumor 313 is on the same irradiation ray as an object having a high radiation absorption rate such as a bone region, not only the ribs 511 in front of the tumor 313 but also the spine 315 behind the tumor 313 are subjected to X-ray fluoroscopic measurement, and thus the tumor 313 is not rendered.

Here, regarding the two-dimensional conversion information stored in the image projection unit 123, the image projection unit 123 may project the two-dimensional conversion information with fidelity to the three-dimensional position correlation as illustrated in (b). In this case, the projection method can be implemented by applying a method called volume rendering. Volume rendering method includes a method of displaying the highest value in the visual line direction 502 (MIP: maximum intensity projection), a method of displaying a value obtained by summing voxel values from the side farther from the viewpoint (ray sum), a method of changing the color (pseudo color) or transparency according to the voxel value, and a method of preferentially displaying a portion where the voxel value suddenly changes. Other usable methods include surface rendering, in which a surface is represented by delineating only an isosurface of voxel values.

It is also possible to superimpose only organs in physical contact with the tumor 313 or organs whose surface (boundary) position is closer than a certain position. In either case, as described above, the design is made such that the in-treatment confirmation image 301 itself is not difficult to view.

### <Image Change Example>

The following will describe a method of indicating the degree of change in the body of the subject from the treatment planning time to the treatment execution time. The series of processes described here is implemented by registering the analysis result for the three-dimensional image at the treatment planning time with the image at the treatment execution time. Accordingly, it is possible to quantify the change in time from the treatment planning time to the treatment execution time using the change information at the time of registration.

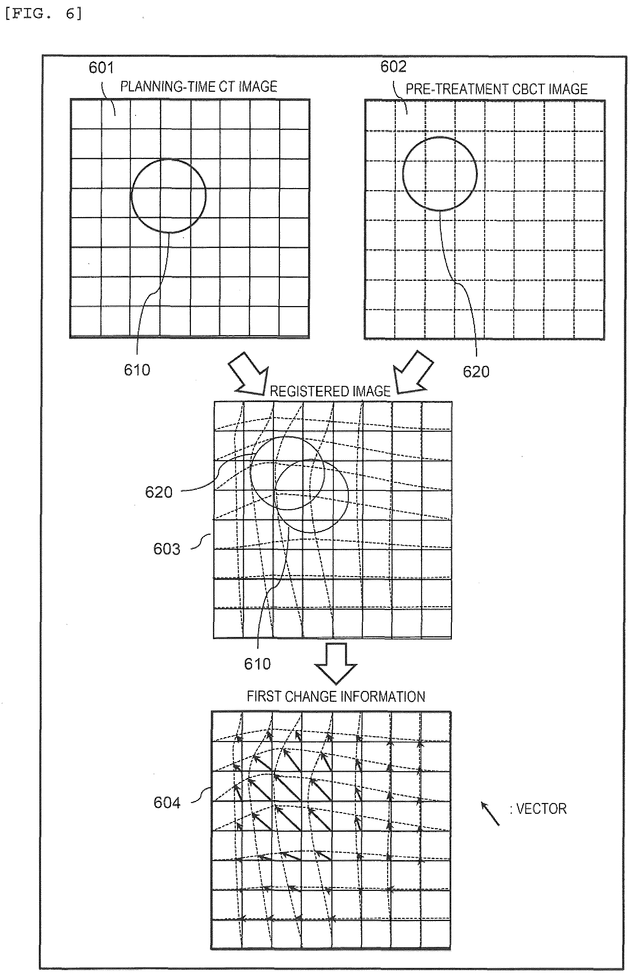

FIG. 6 is a diagram illustrating Registration Example 1. Tissue images 610 and 620 simulating a certain tissue rendered in a planning-time CT image 601 and a pre-treatment CBCT image 602 are included. Each intersection point (grid point) of the grid of the planning-time CT image 601 and the pre-treatment CBCT image 602 indicates a coordinate point. Compared to the tissue image 610 of the planning-time CT image 601, the tissue image 620 of the pre-treatment CBCT image 602 is located at the upper left.

A registered image 603 is obtained by the relation analysis unit 122 registering the planning-time CT image 601 and the pre-treatment CBCT image 602 at the same coordinate point. In the registered image 603, each coordinate point of the planning-time CT image 601 is fixed. On the other hand, the position of each coordinate point of the pre-treatment CBCT image 602 is shifted from the position of each coordinate point of the planning-time CT image 601 according to the movement component from the tissue image 610 to the tissue image 620, and the grids of the pre-treatment CBCT image 602 are distorted according to the displacement.

The relation analysis unit 122 calculates the first change information 604 based on the registered image 603. The first change information 604 is represented by a vector between each coordinate point of the planning-time CT image 601 and each coordinate point of the pre-treatment CBCT image 602 in which the position of the grid is shifted according to the position shift. That is, the vector is information indicating where each coordinate point of the planning-time CT image 601 moves after the registration.

In order to quantitatively estimate the degree of movement in the body of the subject, the relation analysis unit 122 first executes rigid registration without deformation on the planning-time CT image 601 and the pre-treatment CBCT image 602. Thereafter, the relation analysis unit 122 calculates the first change information 604 as shown here for the result of non-rigid registration.

The relation analysis unit 122 calculates, for example, the sum or mean displacement of the vectors of the coordinate points as the first change information 604. Further, the relation analysis unit 122 may determine whether the first change information 604 is equal to or greater than a threshold set in advance, or may normalize the first change information 604 with a value between 0 and 1. The result of such determination or normalization indicates the likelihood of registration and thus is referred to as estimated likelihood. If it is desired to individually determine the first change information 604 for each tissue, the relation analysis unit 122 may calculate the first change information 604 for each tissue.

The generation unit 124 may superimpose the estimated likelihood on the region of the in-treatment confirmation image together with the contour of the region analyzed by the region analysis unit 121.

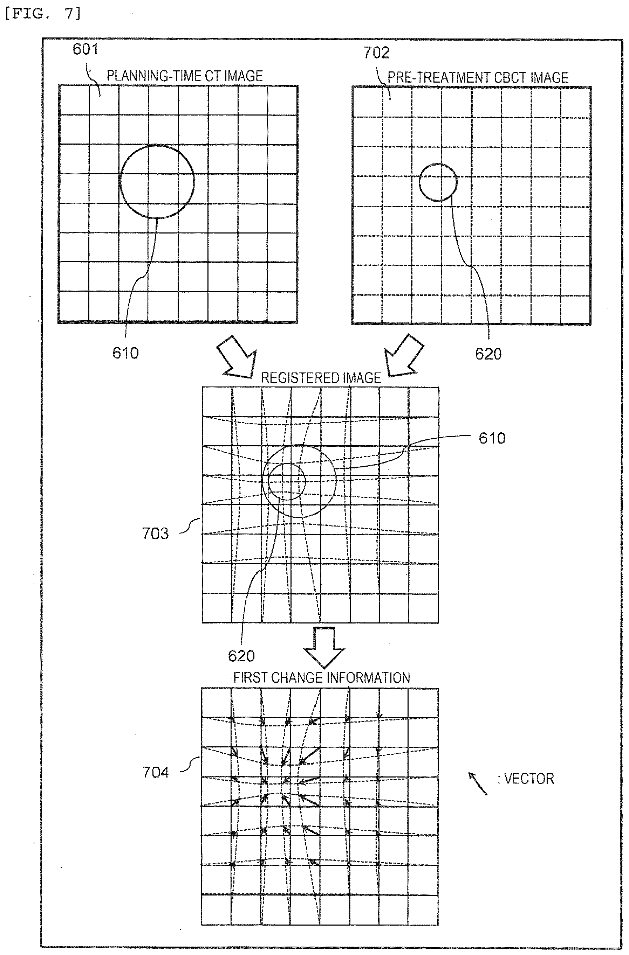

FIG. 7 is a diagram illustrating Registration Example 2. In Registration Example 2, the pre-treatment CBCT image 702 is an image captured when the tissue contracts, and the size of the tissue image 620 is smaller than that in FIG. 6. For example, if the tissue rendered as the tissue images 610 and 620 is a tumor, the tumor size is reduced due to the effect of the treatment in the process of the treatment. Similarly to Registration Example 1, the relation analysis unit 122 generates a registered image 703 by registering the planning-time CT image 601 and the pre-treatment CBCT image 702, and calculates the first change information 704 based on the registered image 703.

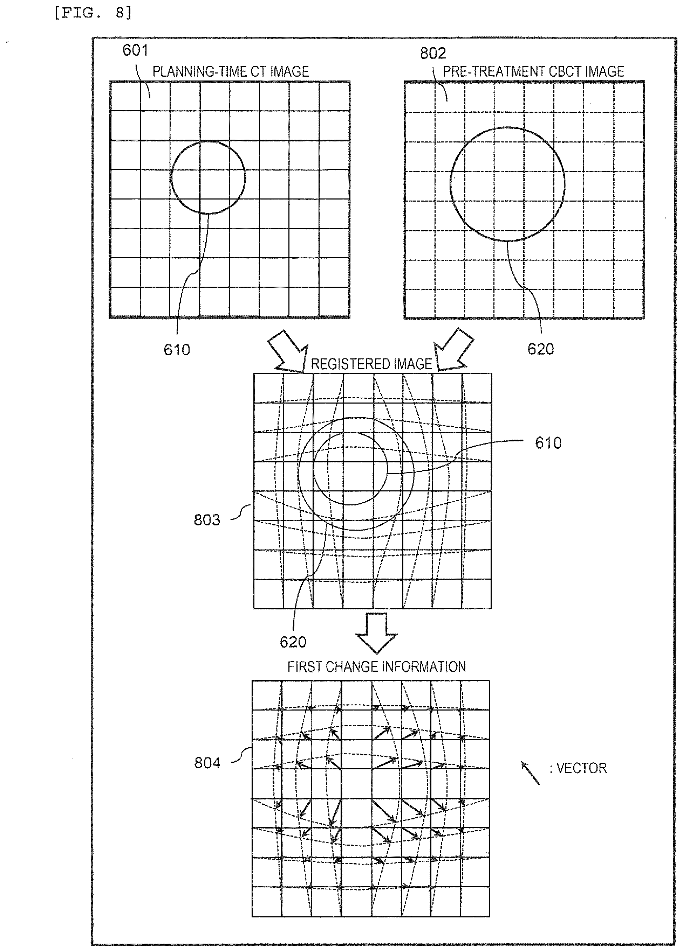

FIG. 8 is a diagram illustrating Registration Example 3. In Registration Example 3, the pre-treatment CBCT image 802 is an image captured when the tissue expands, and the size of the tissue image 620 is larger than that in FIG. 6. For example, if the tissue rendered as the tissue images 610 and 620 is a tumor, the tumor size is reduced if the tissue deteriorates between the planning time and the treatment time. Similarly to Registration Examples 1 and 2, the relation analysis unit 122 generates a registered image 803 by registering the planning-time CT image 601 and the pre-treatment CBCT image 802, and calculates the first change information 804 based on the registered image 803.

As illustrated in FIGS. 7 and 8, it is considered that the directions of the vectors of the coordinate points after the registration become a radial shape. Such a case causes cancellation and thus cannot indicate the change in size of the tumor if according to the summation or averaging of the vectors as in Registration Example 1 of FIG. 6. Therefore, in Registration Examples 2 and 3 illustrated in FIGS. 7 and 8, the relation analysis unit 122 can quantify the change in size by using the sum or mean of the sizes of the vectors as a quantitative value. Although it can also be considered that the size may change or move, both the size change and the movement can be quantified by using both the sum or mean of the vectors and the sum or mean of the sizes.

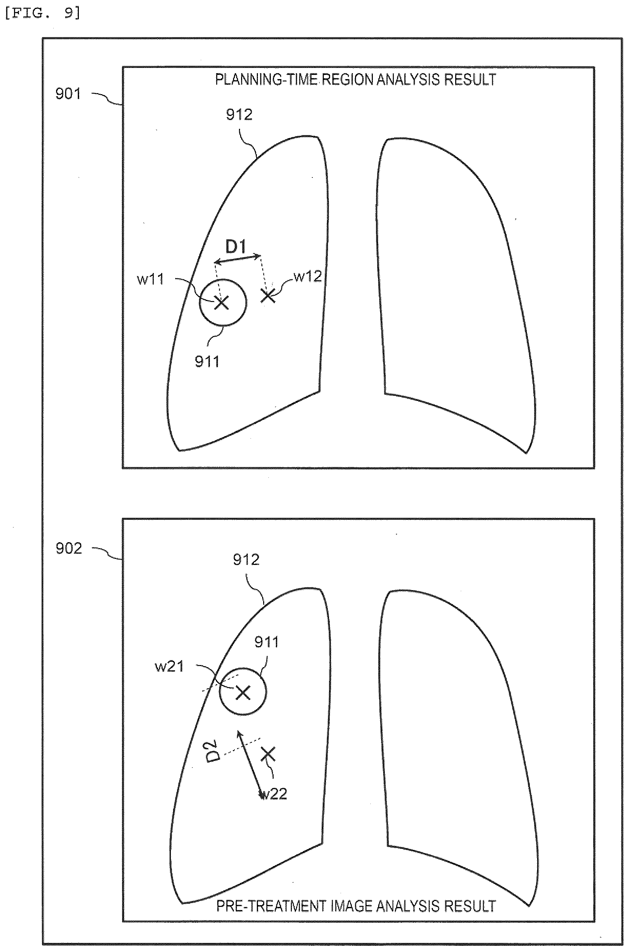

FIG. 9 is a diagram illustrating Registration Example 4. FIG. 9 illustrates an example in which the position correlation between the tumor and each organ is quantified after the registration. That is, this is an example of a case where the relative change amount (distance) of the organs or the tumor is quantified instead of the absolute change amount (movement amount) of the organs or the tumor as illustrated in FIG. 6. The region analysis unit 121 does not execute region analysis on the pre-treatment CBCT image, but information equivalent to the region analysis result by the region analysis unit 121 can be obtained by the registration by the relation analysis unit 122. In Registration Example 4, in each of the planning-time region analysis result 901 and the pre-treatment image analysis result 902, the relation analysis unit 122 may obtain the centroid of the tumor and the centroid of the region of each tissue, and use the inter-centroid distance as an index for quantifying the position correlation between the tumor and each tissue.

Specifically, for example, the inter-centroid distance between a centroid w11 of a tumor 911 and a centroid w12 of a right lung 912 in the planning-time CT image 901 is D1, and the inter-centroid distance between a centroid w21 of the tumor 911 and a centroid w22 of the right lung 912 in the pre-treatment image analysis result 902 is D2. By calculating the absolute value | D1 - D2 | of the difference between the inter-centroid distance D1 and the inter-centroid distance D2, the relation analysis unit 122 can calculate the displacement, which is a change in the position correlation, as the first change information.

Also in this case, the relation analysis unit 122 may determine whether the absolute value | D1 - D2 | of the difference is equal to or greater than a threshold set in advance, or may normalize the absolute value | D1 - D2 | of the difference with a value between 0 and 1. Such determination result and normalization result are also the estimated likelihood described above.

Therefore, the generation unit 124 may superimpose the estimated likelihood on the region of the in-treatment confirmation image together with the contour of the region analyzed by the region analysis unit 121. This enables quantitative indication of the position correlation between the tumor and the tissue, instead of the change amount in each tissue.

### <Visualization of Estimated Likelihood of Tumor Position>

The following will describe an example of visualizing the estimated likelihood of the tumor position. For example, with respect to the tumor position estimated in the process illustrated in FIG. 2, the irradiated position confirmation support device 102 can calculate and superimpose the estimated likelihood by determining the degree of change of the tumor position from the planning time point as in the indices described in FIGS. 6 to 9.

In addition, the irradiated position confirmation support device 102 can also adopt a method of estimating the position of a tumor by means different from the processing illustrated in FIG. 2. For example, region analysis on the pre-treatment CBCT image may be possible. The irradiated position confirmation support device 102 can also determine the difference in the result when the position is estimated by a plurality of methods (including the process of FIG. 2), and determine that the estimated likelihood is low if the difference is large and that the estimated likelihood is high if the difference is small. The generation unit 124 can change the contour type in accordance with the magnitude of the estimated likelihood, or can display the value of the estimated likelihood as characters in a superimposed manner.

Although it is difficult to guarantee the accuracy if the tumor is not rendered on the pre-treatment CBCT image, the estimated position of the tumor is visualized as the likelihood as shown here, and thus can be used for support when the operator determines the validity.

### <Video>

A case where the planning-time CT image and the pre-treatment CBCT image are four-dimensional images, that is, videos (a plurality of time-series images) will be described. For example, it is known that the chest is greatly affected by respiration and a tumor in the lung region may move greatly. In this case, the relation analysis unit 122 registers the images in accordance with the breathing cycle periodically changing in the body, such as the planning-time CT image at the expiration time and the pre-treatment CBCT image at the expiration time, and the planning-time CT image at the inhalation time and the pre-treatment CBCT image at the inhalation time.

As a method for acquiring the breathing cycle, when acquiring the planning-time CT image (video), the pre-treatment CBCT image (video) and the in-treatment confirmation image (video), a marker is simultaneously attached to the body surface of the subject to capture the movement thereof by a camera, and the acquisition unit 120 acquires waveform data on the breathing cycle of each image. There is a method of analyzing with the timings aligned. In addition, the acquisition unit 120 may grasp the movement of the region of the bone or the like at each time point of the breathing cycle in advance and estimate the breathing cycle time point of each acquired image.

The relation analysis unit 122 synchronizes (matches) the waveform data on the two breathing cycles, and registers images at the same timing. Specifically, for example, in step S202, the relation analysis unit 122 synchronizes the waveform data on the breathing cycle at the time of capturing the planning-time CT image (video) with the waveform data on the breathing cycle at the time of capturing the pre-treatment CBCT image (video), and registers the planning-time CT image and the pre-treatment CBCT image at the same timing.

Similarly, in step S206, the waveform data on the breathing cycle at the time of capturing the in-treatment confirmation image (video) and the waveform data on the breathing cycle at the time of capturing the pre-treatment CBCT image (video) are synchronized, and the in-treatment confirmation image and the pre-treatment CBCT image at the same timing are registered. This enables registration with reduced influence of movement of the tumor due to respiration.

Further, the relation analysis unit 122 can obtain a deformed projection analysis result with higher accuracy by deforming (step S207) the projection analysis result obtained by the registration in which the influence of the movement of the tumor due to respiration is reduced (step S205) with the second change information obtained by the registration in which the influence of the movement of the tumor due to respiration is reduced (step S206).

### <Display Example of Monitor 104>

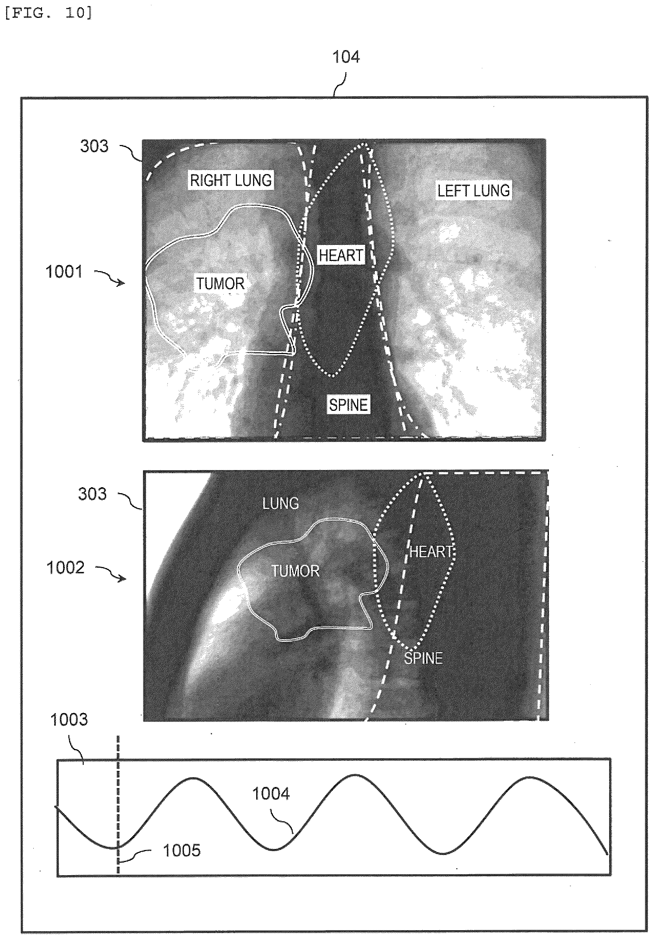

FIG. 10 is a diagram illustrating a display example of the monitor 104. The monitor 104 displays a front superimposed image 1001, a lateral superimposed image 1002, and a graph 1003. The front superimposed image 1001 is a superimposed image 303 of the subject viewed from the front, and the lateral superimposed image 1002 is a superimposed image 303 of the subject viewed from the lateral side.

The graph 1003 illustrates waveform data 1004 on the breathing cycle of the subject. The horizontal axis represents the time, and the vertical axis represents the respiration amplitude (inhalation amount). The waveform data 1004 is waveform data in which the breathing cycle at the time of capturing the planning-time CT image (video) is synchronized with the breathing cycle at the time of capturing the pre-treatment CBCT image (video). The front superimposed image 1001 and the lateral superimposed image 1002 are superimposed images 303 at a breathing timing 1005. When the analysis in consideration of the breathing cycle as described above is executed, it is possible to grasp whether the current time is the expiration time or the inhalation time in the breathing cycle.

The irradiated position confirmation support device 102 does not include a medical imaging device (not illustrated) in the above-described embodiment, but the irradiated position confirmation support device 102 may include a medical imaging device, and the irradiated position confirmation support device 102 may function as a part of the medical imaging device.

### <Example of Hardware Structure of Irradiated Position Confirmation Support System 100>

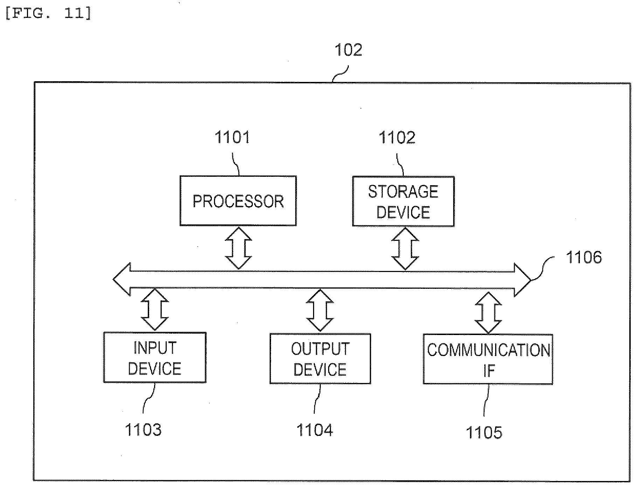

FIG. 11 is a block diagram illustrating a hardware structure example of the irradiated position confirmation support system 100. The irradiated position confirmation support system 100 includes a processor 1101, a storage device 1102, an input device 1103, an output device 1104, and a communication interface (communication IF) 1105. The processor 1101, the storage device 1102, the input device 1103, the output device 1104, and the communication IF 1105 are connected to one another by a bus 1106. The processor 1101 controls the irradiated position confirmation support system 100. The storage device 1102 is a work area of the processor 1101. The storage device 1102 is a non-transitory or transitory recording medium that stores various programs or data. Examples of the storage device 1102 include a read only memory (ROM), a random access memory (RAM), a hard disk drive (HDD), and a flash memory. The input device 1103 inputs data. Examples of the input device 1103 include a keyboard, a mouse, a touch panel, a numeric keypad, a scanner, a microphone, and a sensor. The output device 1104 outputs data. Examples of the output device 1104 include a display, a printer, and a speaker. The communication IF 1105 is connected to a network to transmit and receive data.

The input device 1103 may include the input device 101 illustrated in FIG. 1. The storage device 1102 may include the medical information storage device 103 illustrated in FIG. 1. The output device 1104 may include the monitor 104 illustrated in FIG. 1.

As described above, according to the irradiated position confirmation support device 102, it is possible to support the confirmation of the validity of estimation when presenting an estimated position of a lesion that is invisible or difficult to see due to the angle or the situation on an irradiated position confirmation screen at the time of moving body tracking irradiation.

In the above-described embodiment, the image projection unit 123 performs projection in steps S204 and S205, but may project the three-dimensional planning-time CT image and the three-dimensional pre-treatment CBCT image on a two-dimensional plane before the region analysis unit 121 performs the region analysis.

The invention is not limited to the above embodiments, and includes various modifications and equivalent configurations within the scope of the appended claims. For example, the above embodiment is described in detail for easy understanding of the invention, and the invention is not necessarily limited to those including all the configurations described above. A part of a configuration of one embodiment may be replaced with a configuration of another embodiment. A configuration of one embodiment may also be added to a configuration of another embodiment. Another configuration may be added to a part of a configuration of each embodiment, and a part of the configuration of each embodiment may be deleted or replaced with another configuration.

A part or all of the above configurations, functions, processing units, processing methods, and the like may be implemented by hardware by, for example, designing with an integrated circuit, or may be implemented by software by, for example, a processor interpreting and executing a program for implementing each function.

Information on such as a program, a table, and a file for implementing each function can be stored in a storage device such as a memory, a hard disk, or a solid state drive (SSD), or in a recording medium such as an integrated circuit (IC) card, an SD card, or a digital versatile disc (DVD) .

Control lines and information lines considered to be necessary for description are illustrated, and not all control lines and information lines necessary for implementation are illustrated. Actually, almost all components may be considered to be connected to one another.

## Claims

1. An irradiated position confirmation support device comprising:
an acquisition unit configured to acquire a first medical image acquired by imaging an inside of a body of a subject, a second medical image acquired by imaging the inside of the body of the subject after imaging the first medical image, and a third medical image acquired by imaging the inside of the body of the subject after imaging the second medical image;
a region analysis unit configured to analyze a region included in the first medical image acquired by the acquisition unit;
a relation analysis unit configured to calculate first change information indicating a change related to the same tissue in the first medical image and the second medical image by registering the first medical image with the second medical image acquired by the acquisition unit, and to output a deformed region analysis result by deforming a region analysis result acquired by the region analysis unit based on the first change information; and
a generation unit configured to generate a superimposed image by superimposing the deformed region analysis result acquired by the relation analysis unit on the third medical image acquired by the acquisition unit.

2. The irradiated position confirmation support device according to claim 1, wherein
the region analysis result and the deformed region analysis result include information specifying a shape of the region, and
the generation unit generates the superimposed image by superimposing the information specifying the shape of the region on the third medical image.

3. The irradiated position confirmation support device according to claim 2, wherein
the information specifying the shape of the region is a contour of the region.

4. The irradiated position confirmation support device according to claim 2, wherein
the information specifying the shape of the region is an image in which the region is made translucent.

5. The irradiated position confirmation support device according to claim 1, wherein
the first change information is information indicating displacement of the tissue.

6. The irradiated position confirmation support device according to claim 1, wherein
the first change information is information indicating a change in size of the tissue.

7. The irradiated position confirmation support device according to claim 1, wherein
the region analysis result and the deformed region analysis result include a character string indicating a name of the region, and
the generation unit generates the superimposed image by superimposing the character string on the third medical image.

8. The irradiated position confirmation support device according to claim 7, wherein
the generation unit generates the superimposed image by superimposing the character string on a position of the region indicated by the character string on the third medical image.

9. The irradiated position confirmation support device according to claim 1, wherein
the relation analysis unit calculates, based on the first change information, an estimated likelihood indicating a likelihood of a change in the region caused by the registration between the first medical image and the second medical image, and
the generation unit generates the superimposed image by superimposing the estimated likelihood on the third medical image.

10. The irradiated position confirmation support device according to claim 9, wherein
the generation unit generates the superimposed image by superimposing the estimated likelihood on a position of the region corresponding to the estimated likelihood on the third medical image.

11. The irradiated position confirmation support device according to claim 1, wherein
the relation analysis unit calculates second change information indicating a change related to the same tissue in the second medical image and the third medical image by registering the second medical image with the second medical image, and outputs the deformed region analysis result by deforming the deformed region analysis result based on the second change information.

12. The irradiated position confirmation support device according to claim 1, wherein
the acquisition unit acquires a plurality of time-series first medical images, periodic first change information in the body of the subject at the time of capturing the first medical images, a plurality of time-series second medical images, periodic first change information in the body of the subject at the time of capturing the first medical images, and periodic second change information in the body of the subject at the time of capturing the second medical images, and
the relation analysis unit registers the first medical images and the second medical images at a timing at which the first change information and the second change information are synchronized.

13. An irradiated position confirmation support method to be executed by an irradiated position confirmation support device including a processor configured to execute a program and a storage device storing the program,
the irradiated position confirmation support method comprising the processor executing:
an acquisition processing of acquiring a first medical image acquired by imaging an inside of a body of a subject, a second medical image acquired by imaging the inside of the body of the subject after imaging the first medical image, and a third medical image acquired by imaging the inside of the body of the subject after imaging the second medical image;
a region analysis processing of analyzing a region included in the first medical image acquired by the acquisition processing;
a relation analysis processing of calculating first change information indicating a change related to the same tissue in the first medical image and the second medical image by registering the first medical image with the second medical image acquired by the acquisition processing, and outputting a deformed region analysis result by deforming a region analysis result acquired by the region analysis processing based on the first change information; and
a generation processing of generating a superimposed image by superimposing the deformed region analysis result acquired by the relation analysis processing on the third medical image acquired by the acquisition processing.

14. An irradiated position confirmation support program for causing a processor to execute:
an acquisition processing of acquiring a first medical image acquired by imaging an inside of a body of a subject, a second medical image acquired by imaging the inside of the body of the subject after imaging the first medical image, and a third medical image acquired by imaging the inside of the body of the subject after imaging the second medical image;
a region analysis processing of analyzing a region included in the first medical image acquired by the acquisition processing;
a relation analysis processing of calculating first change information indicating a change related to the same tissue in the first medical image and the second medical image by registering the first medical image with the second medical image acquired by the acquisition processing, and outputting a deformed region analysis result by deforming a region analysis result acquired by the region analysis processing based on the first change information; and
a generation processing of generating a superimposed image by superimposing the deformed region analysis result acquired by the relation analysis processing on the third medical image acquired by the acquisition processing.
